# EUROPEAN PATENT SPECIFICATION

(11) **EP 1 582 598 B1**
(45) Date of publication and mention of the grant of the patent: **23.01.2008**
(21) Application number: 04030193.9
(22) Date of filing: 20.12.2004
(51) Int. Cl.: C12Q 1/68, G01N 21/64, G01N 33/558

(54) **Optoelectronic rapid diagnostic test system**
Optoelektronische Vorrichtung zum schnellen diagnostischen Assay
Système optoélectronique rapid pour tests de diagnostic rapide

(30) Priority: 01.04.2004 US 816636
(43) Date of publication of application: 05.10.2005
(73) Proprietor: Avago Technologies General IP (Singapore) Pte. Ltd, Singapore 768923 (SG)
(72) Inventor: Petruno, Patrick T., San Jose CA 95120 (US); Roitman, Daniel B., Menlo Park CA 94025 (US); Zhou, Rong, Sunnyvale CA 94087 (US); Wilson, Robert E., Palo Alto CA 94303 (US); Chen, Ye, San Jose CA 95120 (US)
(74) Representative: Dilg, Andreas

(56) References cited:
- WO-A-03/098278
- WO-A-20/04003527
- US-A1- 2002 146 844
- US-A1- 2004 018 637

## Description

### BACKGROUND

Rapid diagnostic test kits are currently available for testing for a wide variety of medical and environmental conditions. Commonly, such test kits employ an analyte-specific binding assay to detect or measure a specific environmentally or biologically relevant compound such as a hormone, a metabolite, a toxin, or a pathogen-derived antigen.

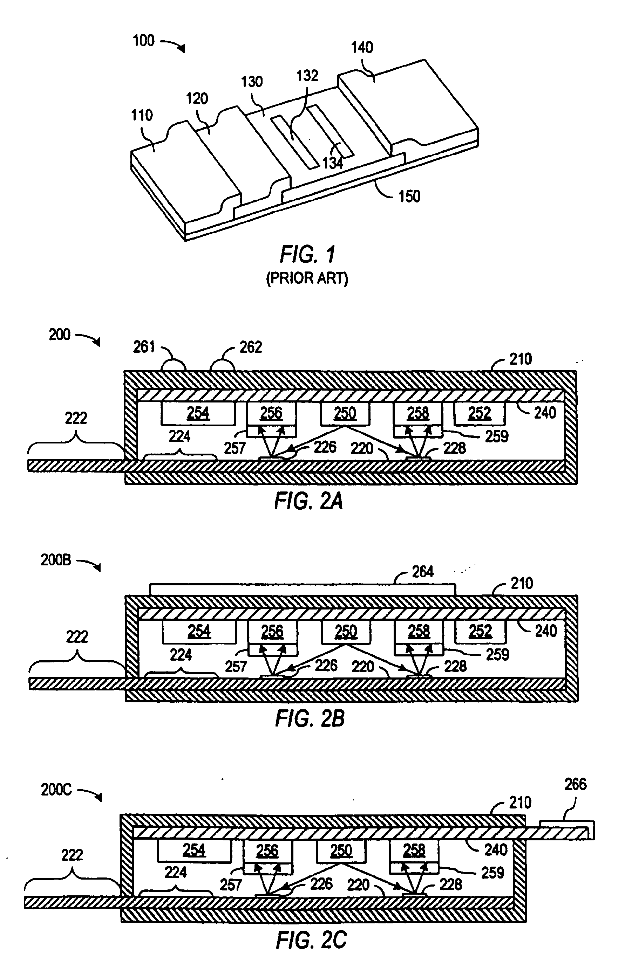

A convenient structure for performing a binding assay is a "lateral flow" strip such as test strip 100 illustrated in Fig. 1. Test strip 100 includes several "zones" that are arranged along a flow path of a sample. In particular, test strip 100 includes a sample receiving zone 110, a labeling zone 120, a capture or detection zone 130, and an absorbent zone or sink 140. Zones 110, 120, 130, and 140, which can be attached to a common backing 150, are generally made of a material such as chemically treated nitrocellulose that allows fluid flow by capillary action.

An advantage of test strip 100 and of a lateral flow immunoassay generally is the ease of the testing procedure and the rapid availability of test results. In particular, a user simply applies a fluid sample such as blood, urine, or saliva to sample receiving zone 110. Capillary action then draws the liquid sample downstream into labeling zone 120, which contains a substance for indirect labeling of a target analyte. For medical testing, the labeling substances are generally immunoglobulin with attached dye molecules but alternatively may be a non-immunoglobulin labeled compound that specifically binds the target analyte.

The sample flows from labeling zone 120 into capture zone 130 where the sample contacts a test region or stripe 132 containing an immobilized compound capable of specifically binding the labeled target analyte or a complex that the analyte and labeling substance form. As a specific example, analyte-specific immunoglobulins can be immobilized in capture zone 130. Labeled target analytes bind the immobilized immunoglobulins, so that test stripe 132 retains the labeled analytes. The presence of the labeled analyte in the sample generally results in a visually detectable coloring in test stripe 132 that appears within minutes of starting the test.

A control stripe 134 in capture zone 130 is useful for indicating that a procedure has been performed. Control stripe 134 is downstream of test stripe 132 and operates to bind and retain the labeling substance. Visible coloring of control stripe 134 indicates the presence of the labeling substance resulting from the liquid sample flowing through capture zone 130. When the target analyte is not present in the sample, test stripe 132 shows no visible coloring, but the accumulation of the label in control stripe 134 indicates that the sample has flown through capture zone 130. Absorbent zone 140 then captures any excess sample.

One problem with these immunoassay procedures is the difficulty in providing quantitative measurements. In particular, a quantitative measurement may require determining the number of complexes bound in test stripe 132. Measuring equipment for such determinations can be expensive and is vulnerable to contamination since capture zone 120, which contains the sample, is generally exposed for measurement. Further, the intensity of dyes used in the test typically degrade very rapidly (e.g., within minutes or hours) when exposed to light, so that quantitative measurements based on the intensity of color must somehow account for dye degradation. On the other hand, a home user of a single-use rapid diagnostic test kit may have difficulty interpreting a test result from the color or shade of test stripe 132, particularly since dye intensity within minutes.

Another testing technology, which is generally performed in laboratories, simultaneously subjects a sample to a panel of tests. For this type of testing, portions of a sample can be applied to separate test solutions. Each test solution generally contains a labeled compound that specifically binds a target analyte associated with the test being performed. Conventionally, the tests are separate because the labeled compounds that bind different target analytes are typically difficult to distinguish if combined in the same solution.

U.S. Pat. No. 6,630,307, entitled "Method of Detecting an Analyte in a Sample Using Semiconductor Nanocrystals as a Detectable Label," describes a process that labels binding compounds for different target analytes with different types of semiconductor nanocrystals or quantum dots. The different types of nanocrystals when exposed to a suitable wavelength of light fluoresce to produce light of different wavelengths. Accordingly, binding compounds labeled with different combinations of quantum dots can be distinguished by spectral analysis of the fluorescent light emitted from the quantum dots.

US 2004/018637 A1 discloses a method for performing a lateral flow assay. The method includes depositing a sample on a test strip at an application region, detecting a first detection signal arising from the test strip in the first detection zone, and generating a baseline for the first measurement zone by interpolating between values of the detection signal outside of the first measurement zone and inside of the first detection zone. The method may include locating a beginning boundary and an ending boundary for the first measurement zone on the test strip. Additional detection zones having measurement zones may also be incorporated with the embodiment.

WO 2004/003527 A relates to methods and apparatus for detecting the presence of analyte in a sample, in particular to luminescence-based methods for such detection.

WO 03/098278 A discloses apparatus and method for differentiating multiple detectable signals by excitation wavelength. The apparatus can include a light source that can emit respective excitation light wavelengths or wavelength ranges towards a sample in a sample retaining region, for example, in a well. The sample can contain two or more detectable markers, for example, fluorescent dyes, each of which can be capable of generating increased detectable emissions when excited in the presence of a target component. The detectable markers can have excitation wavelength ranges and/or emission wavelength ranges that overlap with the ranges of the other detectable markers. A detector can be arranged for detecting an emission wavelength or wavelength range emitted from a first marker within the overlapping wavelength range of at least one of the other markers.

### SUMMARY

In accordance with an aspect of the invention, an optoelectronic rapid diagnostic test system can include a light source such as a light emitting diode (LED) or a laser diode that illuminates a test structure such as a test strip. The test structure preferably uses a persistent fluorescent substance such as a semiconductor nanocrystal or a quantum dot in a labeling substance for a target analyte. The fluorescent substance when bound to the target analyte can be immobilized at a test stripe or region and exposed to light from the light source. The persistent fluorescent substance then fluoresces to emit light of a characteristic wavelength. An electronic photodetector or an imaging device can then detect the light emitted from the test stripe at the characteristic wavelength and generate an electric signal indicating a test result. The test results can be readily quantified since the intensity of the emitted light does not have the rapid time dependence of dyes that are conventionally employed in rapid test systems.

The optoelectronic portion of the diagnostic test kit is inexpensively manufactured for disposable or single-use applications. The electronic nature of the result signal also lends itself to processing and transmission using many electronic systems. For example, control logic in a single-use test module can activate a results indicator (e.g., an external LED or alphanumeric LCD) to unambiguously indicate the test result. Alternatively, a single-use test module can include an interface for connection to reusable data processing equipment. The electronic interface avoids the need for reusable equipment to directly measure or be exposed to materials containing the target analyte and thereby reduces the chance for cross contamination during a sequence of tests.

One specific embodiment of the invention is a rapid diagnostic test system including a photodetector and a light source. The light source illuminates a medium containing a sample, and the photodetector measures light from a test area of the medium when the medium is illuminated.

In one variation of this embodiment of the invention, the medium can be a lateral-flow strip for performing a binding assay and includes a labeling substance that binds a fluorescent structure such as a semiconductor nanocrystal or a quantum dot to a target analyte. The photodetector then measures light having a frequency characteristic of fluorescent light resulting from illuminating the fluorescent structure.

The rapid diagnostic test system can further include a second photodetector, and an optical system positioned to receive light from the test area and direct light to the two photodetectors. In particular, the optical system, which can be implemented using diffractive elements or thin-film color filters, filters or directs different colors of light for separate measurement. For example, the optical system can separate the light having a first frequency from light having a second frequency, direct the light have the first frequency for measurement by the first photodetector, and direct the light have the second frequency for measurement by the second photodetector. With the color separation or filtering, the medium can include a first labeling substance that binds a first fluorescent structure to a first target analyte and a second labeling substance that binds a second fluorescent structure to a second target analyte. When illuminated, the first fluorescent structure emits light having the first frequency, which the first photodetector measures; and the second fluorescent structure emits light having the second frequency, which the second photodetector measures.

The photodetector(s) and the medium can be contained in a single-use module that is either a stand-alone device or that requires connection to a reusable module to complete a test. For example, the reusable module may have a receptacle into which the single-use module is inserted for communication of electrical and/or optical signals.

Another specific embodiment of the invention is a process for rapid diagnostic testing. The test process generally includes: applying a sample to a medium in a single-use module that includes a photodetector; illuminating at least a portion of the medium; and generating an electrical test result signal from the photodetector. The electrical test result signal can be used in a variety of ways to indicate the test result to a user. For example, one variation of the process includes activating a display such as an alphanumeric display or an LED on the single-use module in response to the electrical test result signal. An alternative variation of the process includes outputting the electrical test signal from the single-use module to a reusable module. The reusable module can then implement a user interface that informs a user of the test result.

### BRIEF DESCRIPTION OF THE DRAWINGS

Fig. 1 shows a conventional test strip for an analyte-specific binding assay.

Figs. 2A, 2B, 2C, and 2D show cross-sectional views of optoelectronic rapid diagnostic test kits.

Fig. 3 illustrates a test system using a diffractive optical substrate for focusing and filtering.

Fig. 4 illustrates a test system using refractive lenses and thin-film color filters for optical signals.

Fig. 5 is a cutaway view of a test system containing a battery with a pull-tab for test activation.

Fig. 6 is a perspective view of a test system in which the sample receiving zone of a test strip is inside a case and accessible through an opening in the case.

Figs. 7A and 7B are perspective views of test systems in accordance with embodiments of the invention in which single-use optoelectronic devices have electrical interfaces for communication with reusable test stations.

Use of the same reference symbols in different figures indicates similar or identical items.

### DETAILED DESCRIPTION

Figure 2A to 6 represent background technique useful for understanding the invention.

In accordance with an aspect of the invention, a rapid diagnostic test system employs a disposable optoelectronic device that generates an electronic test result signal. The optoelectronic device preferably contains or is used with a test strip or test structure using a labeling substance that binds a persistent fluorescent substance such as a quantum dot to the target analyte. The test system can include a light source that illuminates a test area with light of the proper wavelength to cause fluorescence and a photodetector such as a photodiode that measures the resulting fluorescent light to detect the target analyte.

Fig. 2A shows a cross-section of a test system 200 where an optoelectronic device reads a test result. System 200 can test for any desired medical or environmental condition or substance including but not limited to glucose, pregnancy, infectious diseases, cholesterol, cardiac markers, signs of drug abuse, chemical contaminants, or biotoxins. System 200 includes a case 210, a test strip 220, and a circuit 240 including a light source 250, a battery 252, a control unit 254, and photodetectors 256 and 258.

Case 210 can be made of plastic or other material suitable for safely containing the liquid sample being analyzed. Case 210 has an opening through which a portion of test strip 220 extends for application of the sample to a sample receiving zone 222 of test strip 220. Alternatively, test strip 220 can be enclosed in case 210, and application of the sample to test strip 220 is through an opening in case 210.

Test strip 220 can be substantially identical to a conventional test strip such as test strip 100 described above in regard to Fig. 1, but in test strip 220, the substance for labeling the target analyte preferably includes a quantum dot or a similar structure that fluoresces at a constant intensity when exposed to light of the proper wavelength. For a test, a user applies a sample to sample receiving zone 222 of test strip 220. The sample flows from receiving zone 222 into a labeling zone 224 inside case 210. The labeling substance binds the quantum dot or other persistent fluorescent structure to the target analyte. The sample including the labeling substance then enters a capture or detection zone that includes a test stripe 226 and a control stripe 228. Test stripe 226 is a region containing an immobilized substance selected to bind and retain the labeled complex containing the target analyte and the quantum dot. Control stripe 228 is a region containing an immobilized substance selected to bind to and retain to the labeling substance.

Light source 250 in circuit 240 illuminates test stripe 226 and control stripe 228 during testing. Light source 250 is preferably a light emitting diode (LED) or a laser diode that emits light of a frequency that causes fluorescence of any quantum dots in test stripe 226 or control stripe 228. Generally, the quantum dots fluoresce under a high frequency (or short wavelength) light, e.g., blue to ultraviolet light, and the fluorescent light has a lower frequency (or a longer wavelength) than the light from light source 250.

Photodetectors 256 and 258 are in the respective paths of light emitted from test stripe 226 and control stripe 228 and measure the fluorescent light from the respective stripes 226 and 228. A baffle or other light directing structure (not shown) can be used to direct light from test stripe 226 to photodetector 256 and light from control strip 228 to photodetector 258. In the embodiment of Fig. 2A, photodetectors 256 and 258 have respective color filters 257 and 259 that transmit light of the frequency associated with the selected fluorescent light but blocks other frequencies, especially the frequency of light emitted from light source 250. Additionally, the labeling substance can include two types of quantum dots. One of the types of quantum dots emits a first wavelength of light and is attached to a substance that binds to the target analyte and to test stripe 226. The other type of quantum dot emits light of a second wavelength and binds to control stripe 228. Color filters 257 and 259 can then be designed so that photodetector 256 measures fluorescent light from the type of quantum dot that test stripe 226 traps when the target analyte is present while photodetector 258 measures fluorescent light from the type of quantum dot that control strip 228 traps.

Quantum dots provide fluorescent light at an intensity that is consistent for long periods of time, instead of rapidly degrading in the way that the intensity of conventional test dyes degrade when exposed to light. As a result, the intensity measurements from detectors 256 and 258, which indicate the amount of fluorescent light, are proportional to the number of quantum dots in the respective stripes 226 and 228 and are not subject to rapid changes with time. These intensity measurements thus provide a quantitative indication of the concentration of the target analyte.

Control unit 254, which can be a standard microcontroller or microprocessor with an analog-to-digital converter, receives electrical signals from detectors 256 and 258. The electric signals indicate the measured intensities from stripes 226 and 228, and control unit 254 processes the electrical test signals and then operates an output system as required to indicate test results. In Fig. 2A, for example, the output system includes LED lights 261 and 263. Control unit 254 can activate one light 261 when the fluorescent light from the test stripe 226 is above a threshold level marking the presence of the target analyte in test stripe 226. Control unit 254 can activate the other light 262 when the intensity of fluorescent light from test stripe 226 is below the threshold level but the intensity that photodetector 258 measures from control stripe 228 is above a threshold level therefore indicating that the sample has passed through test stripe 226. A system with three or more LEDs or particular patterns of flashing of one or more LEDs can similarly indicate other test results (e.g., an inconclusive test) or a test status (e.g., to indicate a test in progress).

Fig. 2B illustrates a test system 200B that is similar to test system 200 but includes an alphanumeric display 264 for output of test results. A two or three character LCD array, for example, could provide numeric output based on the measured intensity of fluorescent light from test stripe 226. Display 264 may be used in conjunction with LEDs such as illustrated in Fig. 2A or other output systems.

Fig. 2C illustrates a test system 200C using yet another test result output technique. In particular, test system 200C outputs an electric signal via external terminals 266 to indicate the test result. As described further below, test system 200C can thus provide the electric test result signal to an electronic device (not shown) that can process, convert, or transmit the test result signal. An advantage of test system 200C is that circuit components such as battery 252 can be removed from circuit 240 to reduce the cost of test system 200C, and power can be supplied to circuit 240 through external terminals 266.

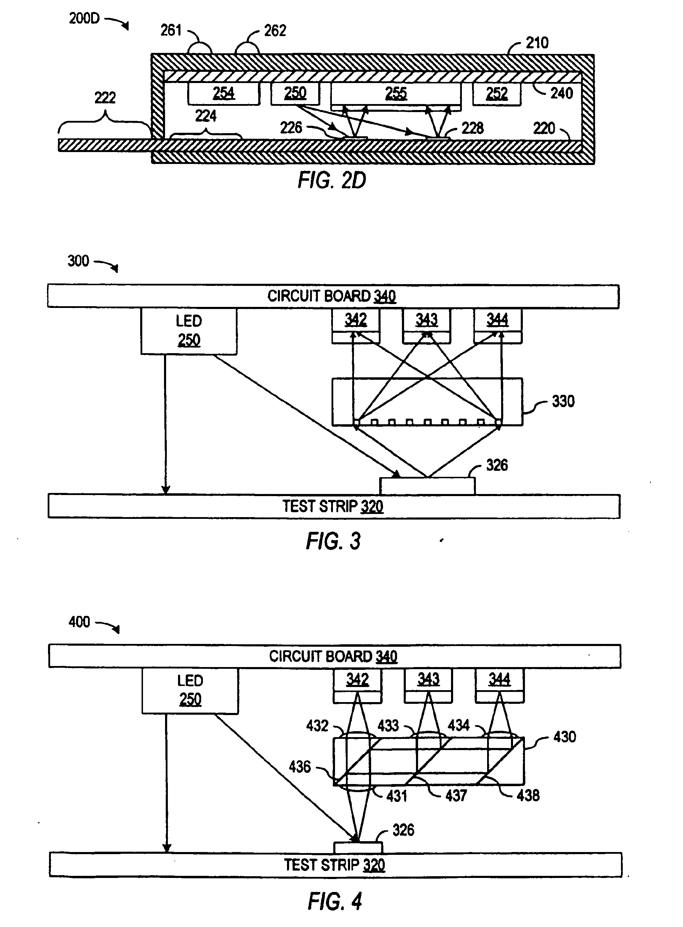

Fig. 2D illustrates a test system 200D that employs an imaging system 255 for detection of fluorescent light. Imaging system 255 can include a two-dimensional CCD or CMOS imaging array or similar optoelectronic imager capable of generating an electronic representation of an image (e.g., an array of pixel values representing a captured image or frame). Control unit 254 can analyze digital images from imaging system 255 to determine the intensity and color of light emitted from stripes 226 and 228. Test results can then be output based on the analysis of the image.

Some advantages of test systems 200, 200B, 200C, and 200D include the ease with which a user receives the test result and the consistency and accuracy of the test results. LED lights 261 and 262 and alphanumeric displays provide results that a user can easily read. In contrast, a conventional rapid diagnostic test relying on a dye to indicate a test result may require that a user distinguish a shade or intensity in a test stripe. This interpretation may be subject to user judgment errors and to dyes that fade within minutes after exposure to light. In contrast, the fluorescence from quantum dots does not fade rapidly with time, and circuit 240 produces a non-subjective and/or quantitative interpretation of the intensity of the fluorescent light.

Another advantage of test systems employing quantum dots is the ability to test for several analytes in the same test stripe. Fig. 3, for example, shows a portion of a test system 300 in accordance with an embodiment of the invention that tests for the presence of multiple target analytes in a sample. Test system 300 includes a test strip 320, an optoelectronic circuit 340, and an intervening optical system 330.

Test strip 320 can be substantially identical to test strip 220, which is described above, but test strip 320 includes multiple labeling substances corresponding to different target analytes. Each labeling substance binds a corresponding type of quantum dot to a corresponding target analyte. The quantum dots for different labeling substances preferably produce fluorescent light having different characteristic wavelengths (e.g., 525, 595, and 655 nm). Suitable quantum dots having different fluorescent frequencies and biological coatings suitable for binding to analyte-specific immunoglobulins are commercially available from Quantum Dot, Inc. Test strip 320 includes a test stripe 326 that is treated to bind to and immobilize the different complexes including the target analytes and respective labeling substances. Testing for multiple analytes in the same test structure is particularly desirable for cholesterol or cardiac panel test system that measures multiple factors.

Light source 250 illuminates test stripe 326 with light of a wavelength that causes all of the different quantum dots to fluoresce. Fluorescent light from test strip 326 will thus contain fluorescent light of different wavelengths if more than one of the target analytes are present in test strip 326. Optical system 330 separates the different wavelengths of light and focuses each of the different wavelengths on a corresponding photodetector 342, 343, or 344. Photodetectors 342, 343, and 344, which can further include appropriate color filters, thus provide separate electrical signals indicating the number of quantum dots of the respective types in test stripe 326 and therefore indicate concentrations of the respective target analytes. Control and output circuits (not shown) can then provide the test results to a user or a separate device as described above in regards to Figs. 2A, 2B, and 2C.

Optical system 330 in Fig. 3 is an optical substrate providing diffractive focusing of the different wavelengths on different photodetectors 342, 343, and 344. In one embodiment of the invention, optical system 330 includes an optical substrate of a material such as glass or plastic with opaque regions or surface discontinuities in a pattern that provides a desired separation or focusing of the different fluorescent wavelengths. However, diffractive optical elements such as optical system 330 can be fabricated inexpensively using other processes and structures.

Fig. 4 shows a portion of test system 400 that is similar to test system 300 of Fig. 3, but test system 400 includes an optical system 430 formed from refractive lenses 431, 432, 433, and 434 and thin-film color filters 436, 437, and 438 on prisms. In particular, lens 431 receives and collimates fluorescent light emitted from test stripe 326 when light source 250 illuminates quantum dots in test stripe 326. Color filter 436 is designed to transmit light of a frequency corresponding to the quantum dots that photodetector 342 measures and to reflect light of the frequency emitted by light source 250 or resulting from fluorescence of other types of quantum dots. Thin films that transmit light of the desired wavelength but reflect light of the other wavelengths can be designed and constructed from a stack of dielectric layers having thicknesses and refractive indices that achieve the desired characteristics. Alternatively, color filter 436 could include a diffractive index grating filter or a colored material. Lens 432 focuses the light transmitted through filter 436 onto the photosensitive area of detector 342, which can include a further color filter for additional selectivity to the desired color of light.

Light reflected from filter 436 is incident on filter 437. Filter 437 is designed to reflect light of the wavelength corresponding to detector 343 and transmit the unwanted wavelengths. Lens 433 focuses the light reflected from filter 437 onto the photosensitive area of detector 343. Light transmitted through filter 437 is incident of filter 438, which is designed to reflect light of the wavelength corresponding to detector 344 and transmit the unwanted wavelengths. Lens 434 focuses the light reflected from filter film 438 onto the photosensitive area of detector 344.

Optical systems 330 and 430 merely provide illustrative examples of an optical system using diffractive elements or thin-film filters for separating different wavelengths of light for measurements. Optical systems using other techniques (e.g., a chromatic prism) could also be employed to separate or filter the fluorescent light. The characteristics and geometry of such optical systems will generally depend on the number of different types of quantum dots used and the wavelengths of the fluorescent light.

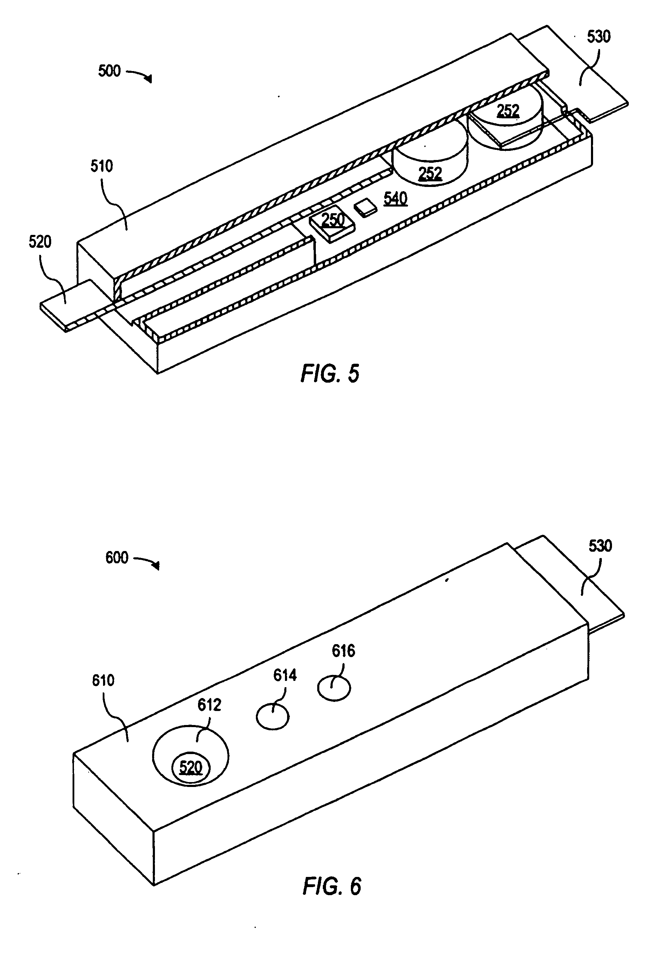

Fig. 5 illustrates a cutaway view of a test system 500. As illustrated, test system 500 includes a case 510 having a first slot at one end through which a test strip 520 extends and second slot at the opposite end through which a pull tab 530 extends. An optoelectronic circuit 540 including a light source 250, batteries 252, and other desired circuit elements is enclosed inside case 510. An optical system (not shown) may additionally be included in case 510 for separation of optical signals or for focusing light onto one or more photodetectors in circuit 540.

Test strip 520 can be substantially identical to test strip 220 or 320, which are described above for measuring one or more target analytes. Pull tab 530 acts as a switch and is initially between a battery 252 and a contact that connects battery 252 to provide power to circuit 540. For testing, a user applies a sample to the exposed portion of test strip 520 and pulls tab 530 out of case 510 to activate circuit 540. Circuit 540 then illuminates test strip 520, measures the intensity of the resulting fluorescence from a target area of test strip 520, and generates an output signal.

Fig. 6 illustrates a test system 600 that encloses a test strip 520 inside a case 610. For sample introduction, case 610 includes an opening 612 that funnels the sample onto a sample receiving zone of test strip 520. An advantage of case 610 is an improved isolation of test strip 520 after introduction of the sample. A cap (not shown) can then be used to cover opening 612 to further improve isolation of the sample during handling of test system 600 after introduction of the sample. Fig. 6 also illustrates that test system 600 can include a pull tab 530 for beginning the electrical operation of test kit 600 and external LEDs 614 and 614 for indication of test status and results.

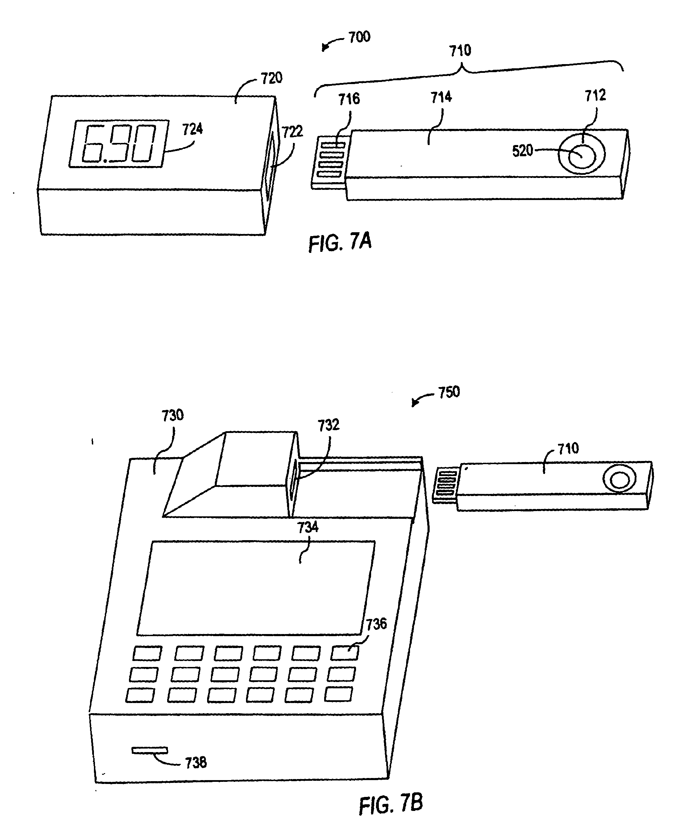

Fig. 7A illustrates a test system 700 in accordance with an embodiment of the invention. Test system 700 includes a single-use module 710 and a reusable module 720. Single-use module 710 includes a test strip 520 that is accessible through an opening 712 in a case 714 in a manner similar to that described above in regard to test system 600 of Fig. 6. Single-use module 710 has an electrical interface including terminals 716 that can be plugged into receptacle 722 of reusable module 720. Reusable module 720 can then display a test result on an LCD display 724 or any other suitable user interface.

Modules 710 and 720 collectively form an optoelectronic circuit capable of reading, analyzing, and providing test results. Generally, single-use module 710 includes one or more photodetectors and optical filters for the fluorescent light generated from test strip 520. The light source is generally in single use module 710 but can alternatively be included in reusable module 720 when single-use module 710 has a window or other optical interface that can convey light of the desired frequency into module 710. Reusable module 720 can include the other circuit elements such as control circuits, batteries, and user interface electronics such as display 724. Through receptacle 722 and terminals 716, reusable module 720 can thus supply power to single-use module 710 and can receive a test result signal. In one embodiment, the test result signal is the analog electric output signals directly from photodetectors in single-use module 710. Alternatively, single-use module 710 can include amplifiers, analog-to-digital converters, and/or other initial signal processing elements that provide a preprocessed signal to reusable module 720.

An advantage of test system 700 is reduction in the cost of the disposable or single-use module 710. In particular, by including more circuit elements in reusable module 720, the cost for repeated tests is decreased and the sophistication of the test result output can be increased (e.g., with alphanumeric or audible output instead of warning lights). This is particularly useful for tests that are repeated such as home testing of glucose levels or almost any diagnostic test performed at a doctor's office. Additionally, reusable module 720 receives an electric signal from single-use module 710 and does not need to directly measure test strip 520 containing a sample. Reusable module 720 is thus not subject to sample contamination that might affect the results of subsequent tests.

Fig. 7B illustrates a test system 750 illustrating using the single-use module 710 with a more elaborate reusable module 730. In the illustrated embodiment, reusable module 730 includes a receptacle 732, a display 734, a keypad 736, and a port 738. Receptacle 732 can be substantially identical to receptacle 722 of Fig. 7A and serves to accommodate single-use module 710 for transmission of power and signals between the two modules 730 and 710. Display 734, which can be an LCD display, a touch screen, or a similar device, is part of the user interface of reusable module 730 and can display any desired information including test results and control information. Keypad 736 provides a user interface for input of data or system control parameters. Port 738 provides a connection to other systems such as a computer or communication network and can be, for example, a jack for modem communications via telephone lines, USB or fire wire, or Ethernet standards to name a few.

Whether a test system employs a relatively simple reusable module 720 as in system 700 of Fig. 7A or a more complex reusable module 730 as shown in Fig. 7B will generally depend on the type of testing performed and the results needed. For example, a commercial product might include one reusable module 720 with a set of six or more single-use modules 710 with the intent being that reusable module 720 is used only a limited number of times (e.g., just with the single use modules in the same package). In contrast, reusable module 730 may be intended for use in a large number of tests and sold separately from single-use modules 710. Further, single-use modules that perform different tests may be made compatible with a standardized reusable module 730, which would permit use of reusable module 730 in a doctor's office, for example, for control and output of many different types of tests.

## Claims

1. A rapid diagnostic test system comprising:
a light source (250) for illuminating a medium (326) containing a sample under test, wherein the medium (326) comprises a labeling substance that binds a persistent fluorescent structure to a target analyte; and
a first photodetector (342) positioned to measure light from a test area (226) of the medium (326),
**characterized in that** the first photodetector (342) and the medium (326) are contained in a single-use module (710).

2. The system of claim 1, wherein the persistent fluorescent structure comprises a quantum dot.

3. The system of claim 1 or 2, further comprising:
a second photodetector (344); and
an optical system (330) positioned to receive light from the test area (326), wherein the optical system (330) separates light having a first frequency from light having a second frequency so that the first photodetector (342) measures light having the first frequency and the second photodetector (344) measures light having the second frequency.

4. The system of claim 1, 2, or 3, wherein the first photodetector comprises a portion of an imaging array (255) that captures an image containing the test area (226) of the medium (220).

5. The system of claim 4, further comprising a reusable module (720) having a receptacle (722) into which the single-use module (710) can be inserted for communication of test signals between the single-use module (710) and the reusable module (720), wherein the reusable module (720) implements a user interface capable of indicating a test result.

6. A process for rapid diagnostic testing, comprising:
applying a sample to a medium (220) containing a labeling substance that binds a persistent fluorescent structure to a target analyte;
illuminating at least a portion of the medium (220); and
generating an electrical signal indicating a detection of fluorescent light, .
**characterized in that** the medium (220) is in a single-use structure (710) that includes a photodetector (256) that measures fluorescent light from the persistent fluorescent structure.

7. The process of claim 6, wherein the persistent fluorescent structure comprises a quantum dot.

8. The process of claim 7, further comprising:
generating an electrical test result signal from the photodetector (256) in the single-use module (710);
transmitting the electrical test result signal from the single-use module (710) to a reusable module (720); and
indicating a test result on the reusable module (720).

## Patentansprüche

1. Schnelldiagnose-Testsystem, aufweisend:
- eine Lichtquelle (250) zum Beleuchten eines Mediums (326), das eine Testprobe enthält, wobei das Medium (326) eine Labelsubstanz aufweist, die eine persistente fluoreszierende Struktur an ein Zielanalyt bindet; und
- einen ersten Photodetektor (342), der positioniert ist, um Licht von einem Testbereich (226) des Mediums (326) zu messen,
**dadurch gekennzeichnet, dass** der erste Photodetektor (342) und das Medium (326) in einem Einwegmodul (710) enthalten sind.

2. System nach Anspruch 1, wobei die persistente fluoreszierende Struktur einen Quantenpunkt aufweist.

3. System nach Anspruch 1 oder 2, ferner aufweisend:
- einen zweiten Photodetektor (344); und
- ein optisches System (330), das angeordnet ist, um Licht aus dem Testbereich (326) zu empfangen, wobei das optische System (330) Licht mit einer ersten Frequenz von Licht mit einer zweiten Frequenz trennt, so dass der erste Photodetektor (342) Licht mit der ersten Frequenz und der zweite Photodetektor (344) Licht mit der zweiten Frequenz misst.

4. System nach Anspruch 1, 2 oder 3, wobei der erste Photodetektor einen Abschnitt eines bilderzeugenden Arrays (255) aufweist, das ein Bild aufnimmt, das den Testbereich (226) des Mediums (220) enthält.

5. System nach Anspruch 4, ferner aufweisend ein wiederverwendbares Modul (720) mit einem Behälter (722), in den das Einwegmodul (710) eingesetzt werden kann, um Testsignale zwischen dem Einwegmodul (710) und dem wiederverwendbaren Modul (720) zu übertragen, wobei das wiederverwendbare Modul (720) eine Benutzerschnittstelle ausbildet, die ein Testergebnis angeben kann.

6. Verfahren zum Schnelldiagnosetesten, aufweisend:
- Zuführen einer Probe zu einem Medium (220), das eine Labelsubstanz enthält, die eine persistente fluoreszierende Struktur an ein Zielanalyt bindet;
- Beleuchten zumindestens eines Abschnitts des Mediums (220); und
- Erzeugen eines elektrischen Signals, das eine Erfassung von fluoreszierendem Licht angibt,
**dadurch gekennzeichnet, dass** das Medium (220) sich in einer Einwegstruktur (710) befindet, die einen Photodetektor aufweist (256), der fluoreszierendes Licht der persistenten fluoreszierenden Struktur misst.

7. Verfahren nach Anspruch 6, wobei die persistente fluoreszierende Struktur einen Quantenpunkt aufweist.

8. Verfahren nach Anspruch 7, ferner aufweisend:
- Erzeugen eines elektrischen Testergebnissignals aus dem Photodetektor (256) in dem Einwegmodul (710);
- Übertragen des elektrischen Testergebnissignals von dem Einwegmodul (710) zu einem wiederverwendbaren Modul (720); und
- Angeben eines Testergebnisses auf dem wiederverwendbaren Modul (720).

## Revendications

1. Système de test de diagnostic rapide comprenant :
■ une source de lumière (250) destinée à illuminer un milieu (326) contenant un échantillon sous test, le milieu (326) comprenant une substance de marquage qui assure la liaison d'une structure fluorescente persistante à un échantillon cible à analyser; et
■ un premier détecteur photosensible (342) positionné pour mesurer la lumière issue d'une surface à observer (226) du milieu (326),
**caractérisé en ce que** le premier détecteur photosensible (342) et le milieu (326) sont contenus dans un module à usage unique (710).

2. Système selon la revendication 1, dans lequel la structure fluorescente persistante comprend un point quantique.

3. Système selon la revendication 1 ou 2, comprenant en outre :
■ un deuxième détecteur photosensible (344) ; et
■ un système optique (330) positionné pour recevoir la lumière issue de la surface à observer (326), le système optique (330) séparant une lumière ayant une première fréquence d'une lumière ayant une deuxième fréquence de telle sorte que le premier détecteur photosensible (342) mesure la lumière ayant la première fréquence et que le deuxième détecteur photosensible (344) mesure la lumière ayant la deuxième fréquence.

4. Système selon la revendication 1, 2, ou 3, dans lequel le premier détecteur photosensible comprend une partie d'un réseau d'imagerie (255) qui capture une image contenant la surface à observer (226) du milieu (220).

5. Système selon la revendication 4, comprenant en outre un module réutilisable (720) comportant un réceptacle (722) dans lequel le module à usage unique (710) peut être inséré pour la communication de signaux de test entre le module à usage unique (710) et le module réutilisable (720), le module réutilisable (720) mettant en oeuvre une interface utilisateur capable d'indiquer un résultat de test.

6. Processus de test de diagnostic rapide, comprenant :
■ l'application d'un échantillon à un milieu (220) contenant une substance de marquage qui assure la liaison d'une structure fluorescente persistante à un échantillon cible à analyser ;
■ l'illumination d'au moins une partie du milieu (220); et
■ la génération d'un signal électrique indiquant une détection de lumière fluorescente,
**caractérisé en ce que** le milieu (220) est dans une structure à usage unique (710) qui comprend un détecteur photosensible (256) qui mesure la lumière fluorescente issue de la structure fluorescente persistante.

7. Processus selon la revendication 6, dans lequel la structure fluorescente persistante comprend un point quantique.

8. Processus selon la revendication 7, comprenant en outre :
■ la génération d'un signal de résultat de test électrique issu du détecteur photosensible (256) dans le module à usage unique (710) ;
■ la transmission du signal de résultat de test électrique issu du module à usage unique (710) vers un module réutilisable (720) ; et
■ l'indication d'un résultat de test sur le module réutilisable (720).
